(19) **Europäisches Patentamt / European Patent Office / Office européen des brevets**

(11) **EP 4 692 421 A1**

(12) **EUROPEAN PATENT APPLICATION**
published in accordance with Art. 153(4) EPC

(43) Date of publication:
**11.02.2026   Bulletin 2026/07**

(21) Application number: **25762018.7**

(22) Date of filing: **27.02.2025**

(51) International Patent Classification (IPC):
**C25B 1/01** (2021.01)        **C01B 32/914** (2017.01)
**C07C 1/32** (2006.01)        **C07C 9/04** (2006.01)
**C07C 9/06** (2006.01)        **C07C 11/04** (2006.01)
**C07C 11/06** (2006.01)        **C07C 11/08** (2006.01)
**C07C 11/22** (2006.01)        **C07C 11/24** (2006.01)
**C25B 1/02** (2006.01)        **C25B 1/135** (2021.01)
**C25B 9/00** (2021.01)        **C25B 9/09** (2021.01)

(52) Cooperative Patent Classification (CPC):
**C01B 32/914; C07C 1/32; C07C 9/04; C07C 9/06;
C07C 11/04; C07C 11/06; C07C 11/08; C07C 11/22;
C07C 11/24; C25B 1/01; C25B 1/02; C25B 1/135;
C25B 9/00; C25B 9/09**

(86) International application number:
**PCT/JP2025/007004**

(87) International publication number:
**WO 2025/183125 (04.09.2025 Gazette 2025/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.02.2024   JP 2024028876
25.11.2024   JP 2024204704**

(71) Applicants:
• **Daikin Industries, Ltd.
Osaka-shi, Osaka 530-0001 (JP)**
• **The Doshisha
Kyoto-shi, Kyoto 602-8580 (JP)**

(72) Inventors:
• **GOTO, Takuya
Kyotanabe-shi, Kyoto 610-0394 (JP)**

• **WATANABE, Takashi
Kyotanabe-shi, Kyoto 610-0394 (JP)**
• **SUZUKI, Yuta
Kyotanabe-shi, Kyoto 610-0394 (JP)**
• **TANAKA, Seiya
Kyotanabe-shi, Kyoto 610-0394 (JP)**
• **FUKUHARA, Hijiri
Kyotanabe-shi, Kyoto 610-0394 (JP)**
• **ISOGAI, Tomohiro
Osaka-Shi, Osaka 530-0001 (JP)**
• **TSUCHII, Takane
Osaka-Shi, Osaka 530-0001 (JP)**
• **YAMAUCHI, Akiyoshi
Osaka-Shi, Osaka 530-0001 (JP)**
• **KISHIKAWA, Yosuke
Osaka-Shi, Osaka 530-0001 (JP)**

(74) Representative: **Hoffmann Eitle
Patent- und Rechtsanwälte PartmbB
Arabellastraße 30
81925 München (DE)**

(54) **METHOD FOR PRODUCING METAL CARBIDE AND HYDROCARBON, AND METAL-CONTAINING MEMBER**

(57)    A method for producing a metal carbide, comprising: preparing a molten salt containing a carbonate ion; preparing an electrode containing a first metal; and applying a voltage to the molten salt using the electrode to obtain a metal carbide composition containing a carbide of the first metal.

EP 4 692 421 A1

## FIG. 1

```
          ┌──────────┐
          │  START   │
          └──────────┘
                │
                ▼
  ┌─────────────────────────────┐
  │ PREPARATION OF FIRST MOLTEN  │─── S11
  │ SALT CONTAINING CARBONATE    │
  │           ION                │
  └─────────────────────────────┘
                │
                ▼
  ┌─────────────────────────────┐
  │   PREPARATION OF FIRST       │─── S12
  │ ELECTRODE CONTAINING FIRST   │
  │           METAL              │
  └─────────────────────────────┘
                │
                ▼
  ┌─────────────────────────────┐
  │   APPLICATION OF VOLTAGE     │─── S13
  │ (PRODUCTION OF FIRST METAL   │
  │          CARBIDE)            │
  └─────────────────────────────┘
                │
                ▼
          ┌──────────┐
          │   END    │
          └──────────┘
```

**Description**

TECHNICAL FIELD

The present invention relates to methods for producing a metal carbide and a hydrocarbon, and a metal-containing member.

BACKGROUND ART

Aluminum carbide has a high hardness and a high melting point and is an industrially important substance. Aluminum carbide is utilized as a filler for a metal material or a resin material, for example, for improving the shear strength and other properties of polishing or composite materials. As a method for producing aluminum carbide, a method for heating metal aluminum or aluminum oxide and carbon in an arc furnace at a high temperature (e.g., 1,100 to 1,800°C) is known. Also, as the method for producing aluminum carbide, a method for bringing ammonia gas into contact with aluminum oxide and carbon heated to a high temperature (e.g., 1,000°C or more) (see Patent Document 1), a method for heating organometallic aluminum such as $Al(CH_3)_3$ or $Al(C_2H_5)_3$ at 950 to 1,100°C (see Non Patent Document 1), and a method for bringing metal aluminum into contact with a lower hydrocarbon at 500 to 900°C (see Patent Document 2) are proposed.

PRIOR ART DOCUMENTS

PATENT DOCUMENTS

    Patent Document 1: JP H1-183411 A
    Patent Document 2: JP 2001-58810 A

NON PATENT Document

Non Patent Document 1: Kiyoshi Itatani, Akira Kishioka, "Some Properties of Aluminum Carbide and Its Related Compounds" Inorganic Materials, 1997, vol. 4, No. 271, p. 633-641

SUMMARY OF THE INVENTION

PROBLEMS TO BE SOLVED BY THE INVENTION

In the methods of Patent Document 1 and Non Patent Document 1, raw materials are heated at high temperatures, so that the energy efficiency is low. In addition, when carbon is used as a raw material, impurities derived from carbon (such as phosphorus and sulfur) contaminate the metal carbide to be obtained, and the purity is reduced. Further, utilization of carbon derived from fossil fuel as a raw material is against decarbonization. The method of Patent Document 2 is undesirable because it requires handling flammable ammonia gas, hydrocarbon gas, and organometallic compounds at high temperatures.

An object of the present disclosure is to provide a production method in which the reaction rapidly proceeds at a relatively low temperature (e.g., 800°C or less) and which is capable of efficiently obtaining a metal carbide without requiring use of a flammable raw material. The present disclosure further provides a method for producing a hydrocarbon from the obtained metal carbide. In addition, the present disclosure provides a metal carbide-supporting metal-containing member.

SOLUTIONS TO PROBLEMS

The present disclosure includes the following aspects.

    [1] A method for producing a metal carbide, comprising:

        preparing a molten salt containing a carbonate ion;
        preparing an electrode containing a first metal; and
        applying a voltage to the molten salt using the electrode to obtain a metal carbide composition containing a carbide of the first metal.

[2] The method for producing a metal carbide according to the above [1], wherein the first metal contains at least one selected from the group consisting of aluminum, beryllium, manganese, scandium, yttrium, lanthanum, and cerium.

[3] The method for producing a metal carbide according to the above [1] or [2], wherein the molten salt contains at least one selected from the group consisting of a sodium ion, a lithium ion, a potassium ion, rubidium, and cesium, as a metal ion.

[4] The method for producing a metal carbide according to any one of the above [1] to [3], wherein the molten salt contains at least one selected from the group consisting of a sodium ion, a lithium ion, and a potassium ion, and at least one selected from the group consisting of a calcium ion, a magnesium ion, a strontium ion, and a barium ion, as metal ions.

[5] The method for producing a metal carbide according to any one of the above [1] to [4], wherein the molten salt contains the carbide of the first metal.

[6] The method for producing a metal carbide according to any one of the above [1] to [5], wherein the carbide composition further contains at least one selected from the group consisting of carbon; a simple substance, a halide, a carbonate, an oxide, a hydride, and a peroxide of the first metal; and a simple substance, a halide, a carbonate, an oxide, and a carbide of the second metal constituting the molten salt.

[7] A method for producing a hydrocarbon, comprising:

preparing a molten salt containing a carbonate ion;
preparing an electrode containing a first metal;
applying a voltage to the molten salt using the electrode to obtain a metal carbide composition containing a carbide of the first metal; and
hydrolyzing the carbide of the first metal to obtain gas containing a hydrocarbon.

[8] The method for producing a hydrocarbon according to the above [7], wherein the gas contains methane.

[9] The method for producing a hydrocarbon according to the above [7] or [8], wherein the gas contains methane and at least one selected from the group consisting of ethylene, ethane, acetylene, methylacetylene, propylene, butene, and hydrogen.

[10] A metal-containing member including:

a base containing a first metal; and
a metal carbide composition supported on the base, the metal carbide composition containing a carbide of the first metal.

EFFECTS OF THE INVENTION

The present disclosure can provide a production method in which the reaction rapidly proceeds at a relatively low temperature and which is capable of efficiently obtaining a metal carbide without requiring use of a flammable raw material, a method for producing a hydrocarbon from the obtained metal carbide, and a metal carbide-supporting metal-containing member.

BRIEF DESCRIPTION OF THE DRAWINGS

FIG. 1 is a flow chart of the method for producing a metal carbide according to the present disclosure.
FIG. 2 is a flow chart of the method for producing a hydrocarbon according to the present disclosure.
FIG. 3 is a graph showing a change with time of a current upon energization in the production of the metal carbide in Example 1.
FIG. 4 is a photograph showing the appearance of the working electrode before energization.
FIG. 5 is a photograph showing the appearance of the working electrode after energization in Example 1.
FIG. 6 is a graph showing the results of XRD analysis of the precipitate obtained in Examples 1 to 3.
FIG. 7 is a graph showing the results of the GC analysis of the gases produced in Examples 1 to 3.
FIG. 8 is a graph showing a change with time of a current upon energization in the production of the metal carbide in Example 2.
FIG. 9 is a photograph showing the appearance of the working electrode after energization in Example 2.
FIG. 10 a graph showing a change with time of a current upon energization in the production of the metal carbide in Example 3.
FIG. 11 is a photograph showing the appearance of the working electrode after energization in Example 3.

DESCRIPTION OF EMBODIMENTS

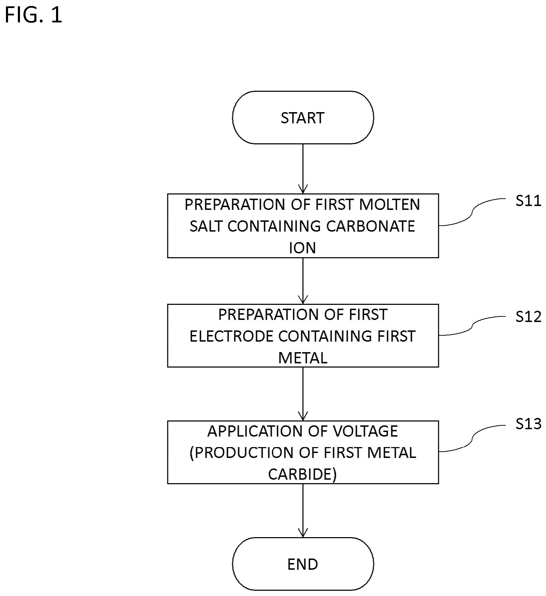

The method for producing a metal carbide according to the present disclosure includes preparing a molten salt containing a carbonate ion, preparing an electrode containing a first metal, and applying a voltage to the molten salt using the electrode to obtain a metal carbide composition containing a carbide of the first metal. Thus, a metal carbide can be efficiently obtained at a relatively low temperature of 800°C or less (e.g., 600°C). FIG. 1 is a flow chart of the method for producing a metal carbide according to the present disclosure.

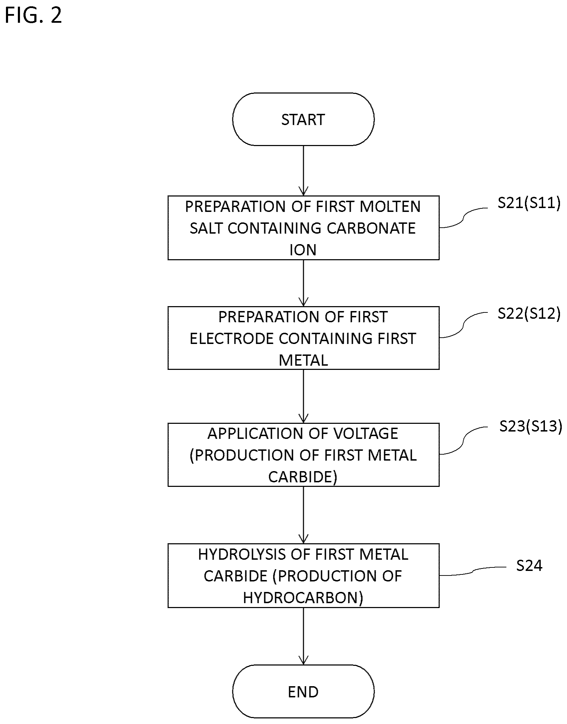

The method for producing a hydrocarbon according to the present disclosure includes preparing a molten salt containing a carbonate ion, preparing an electrode containing a first metal, applying a voltage to the molten salt using the electrode to obtain a metal carbide composition containing a carbide of the first metal, and hydrolyzing the carbide of the first metal to obtain gas containing a hydrocarbon. According to this method, the faradaic efficiency is improved, and a hydrocarbon with high purity can be efficiently obtained. FIG. 2 is a flow chart of the method for producing a hydrocarbon according to the present disclosure.

The metal-containing member according to the present disclosure includes a base containing a first metal, and a metal carbide composition supported on the base, wherein the metal carbide composition contains a carbide of the first metal. This metal-containing member can be utilized in the production of a hydrocarbon.

[Method for producing metal carbide]

In the present embodiment, a carbonate ion derived from carbon dioxide is used. By effectively utilizing $CO_2$, which is said as a cause of global warming, as a carbon source, a carbide composition containing a carbide of the first metal can be obtained.

(i) Preparation of molten salt (S11)

The molten salt containing a carbonate ion derived from carbon dioxide is prepared. The carbonate ion is produced by adding gas containing carbon dioxide to an electrolytic bath. The molten salt also contains a second metal ion. The second metal ion is produced by the electrolysis of the salt of the second metal. In the molten salt, it is not required that the whole second metal salt and carbon dioxide are ionized. In the present embodiment, for convenience, the salt of the second metal contained in the electrolytic bath is referred to as the second metal salt, also in the case where the salt is completely ionized, and the molten salt prepared from the second metal salt and carbon dioxide is referred to as the molten salt, also in the case where the molten salt is not completely ionized.

(Carbonate ion derived from carbon dioxide)

The carbonate ion is produced by adding gas containing carbon dioxide to the electrolytic bath.

Gas containing carbon dioxide (hereinafter, sometimes referred to as $CO_2$ gas) in a gas state is brought into contact with the second metal salt in a liquid state. $CO_2$ gas may be blown into the gas phase part of the electrolytic bath and brought into contact with the liquid surface of the second metal salt, or $CO_2$ gas may be blown into the second metal salt. $CO_2$ gas may be a mixed gas of $CO_2$ and an inert gas (typically, argon). A sufficient amount of $CO_2$ gas may be added to the second metal salt before application of a voltage, or $CO_2$ gas may be added to the second metal salt while applying a voltage.

The amount of $CO_2$ gas blown may be appropriately set depending on the amount of the second metal ion. For example, considering the absorption efficiency of $CO_2$ into the second metal salt, the amount of $CO_2$ gas blown is equivalent to or more than the second metal salt.

From the viewpoint of promoting the dissolution of $CO_2$ into the second metal salt, the diameter of bubbles of the $CO_2$ gas blown is desirably smaller. The diameter of bubbles of the $CO_2$ gas may be 10 mm or less, or may be 1 mm or less. The diameter of bubbles of the $CO_2$ gas may be 100 nm or more, or may be 1 $\mu$m or more. The diameter of bubbles of the $CO_2$ gas is micronized by, for example, bubbling through porous materials made of quartz glass or high purity alumina, stirring with a stirrer, applying vibration, or being irradiated with ultrasonic waves.

$CO_2$ gas is preferably preheated to the temperature of the second metal salt, in advance. Preheating suppresses the reduction in the temperature and solidification of the second metal salt.

(Further anion)

The molten salt may contain an anion other than carbonate ion. A further anion is, for example, at least one selected from the group consisting of a halide ion, a sulfate ion, a phosphate ion, a nitrate ion, an acetate ion, a carboxylate ion, and an oxide ion ($O^{2-}$).

A halide ion may be contained as a further anion. The halide of the second metal is generally used as the molten salt, and is excellent as an electrolyte.

An oxide ion may be contained as a further anion. The oxide ion allows $CO_2$ to be easily ionized.

(Second metal ion)

The second metal ion is, for example, at least one selected from the group consisting of alkali metal ions and alkali earth metal ions. Alkali metal ions and alkali earth metal ions have excellent functions as the electrolyte.

The alkali metal is at least one selected from the group consisting of lithium (Li), sodium (Na), potassium (K), rubidium (Rb), cesium (Cs), and francium (Fr). The alkali metal may be at least one selected from the group consisting of Li, Na, K, Rb, and Cs. In particular, the alkali metal may be at least one selected from the group consisting of Li, Na, K, and Cs.

The alkali earth metal is at least one selected from the group consisting of beryllium (Be), magnesium (Mg), calcium (Ca), strontium (Sr), barium (Ba), and radium (Ra). The alkali earth metal may be at least one selected from the group consisting of Mg, Ca, Sr, and Ba.

In terms of industrial value, the second metal ion may contain an alkali earth metal ion. The second metal ion may contain both an alkali earth metal ion and an alkali metal ion. The alkali metal ion has excellent functions as an electrolyte. For example, the alkali metal ion allows the alkaline earth metal salt to be easily ionized and promotes the production of the alkali earth metal ion, reduces the melting point of the molten salt, and enables electrolysis to be performed at a further low temperature.

The second metal ion may contain at least one selected from the group consisting of Li, Na, K, Rb, and Cs ions as the alkali metal ion, and at least one selected from the group consisting of Be, Mg, Ca, Sr, and Ba ions as the alkali earth metal ion. The second metal ion may contain at least one of Li, Na, and K ions, and a Ca ion.

The second metal ion may contain a further metal ion other than the alkali metal ion and the alkali earth metal ion. As the second metal ion, the same metal ion as the first metal ion may be contained. Examples of the further metal include at least one selected from the group consisting of aluminum (Al), gallium (Ga), indium (In), thallium (Tl), zinc (Zn), cadmium (Cd), gold (Au), silver (Ag), and copper (Cu). Examples of the rare-earth element include scandium (Sc), yttrium (Y), lanthanoid elements, and actinoid elements. The salt of the further metal is preferably ionized at a temperature of 800°C or less.

The amount of the second metal ion contained in the molten salt is not limited. Specific examples of the second metal salt contained in the molten salt include alkali metal halides such as LiF, NaF, KF, RbF, CsF, LiCl, NaCl, KCl, RbCl, CsCl, LiBr, NaBr, KBr, RbBr, CsBr, LiI, NaI, KI, RbI, and CsI; alkaline earth metal halides such as $MgF_2$, $CaF_2$, $SrF_2$, $BaF_2$, $MgCl_2$, $CaCl_2$, $SrCl_2$, $BaCl_2$, $MgBr_2$, $CaBr_2$, $SrBr_2$, $BaBr_2$, $MgI_2$, $CaI_2$, $SrI_2$, and $BaI_2$; rare earth element halides such as $ScCl_3$, $YCl_3$, $LaCl_3$, $CeCl_3$, $PrCl_3$, $NdCl_3$, $PmCl_3$, $SmCl_3$, $EuCl_3$, $GdCl_3$, $TbCl_3$, $DyCl_3$, $HoCl_3$, $ErCl_3$, $TmCl_3$, $YbCl_3$, and $LuCl_3$; earth metal halides such as $AlCl_3$, $GaCl_3$, $InCl_3$, and $TlCl_3$; metal oxides such as $Li_2O$ and $CaO$; metal carbonates such as $Li_2CO_3$, $Na_2CO_3$, and $K_2CO_3$; and metal nitrates such as $LiNO_3$, $NaNO_3$, and $KNO_3$. One of these may be used singly, or two or more may be used in combination. Among them, two or more metal salts may be combined in terms that the melting temperature is easily reduced.

(Additive)

Alternatively, the molten salt may further contain an additive.

Examples of the additive contained in the molten salt include a metal carbide containing the first metal. When the molten salt contains a metal carbide, the faradaic efficiency may be improved. Examples of the metal in the metal carbide include aluminum, beryllium, manganese, scandium, yttrium, lanthanum, and cerium. The metal carbide is preferably $Al_4C_3$, $Be_2C$, $Mn_3C$, $ScC$, $YC$, $Y_2C_3$, $YC_2$, $LaC_2$, or $CeC_2$, and particularly preferably $Al_4C_3$.

(ii) Preparation of electrode containing first metal (S12)

An electrode containing the first metal (hereinafter, sometimes referred to as "metal electrode") is prepared. The metal electrode functions as the cathode upon electrolysis. The first metal contained in the metal electrode is the metal source of the target metal carbide.

The first metal may be present at least on the surface of the metal electrode. The surface of the metal electrode is a part that may be brought into contact with the molten salt.

(First metal)

The first metal is, for example, at least one selected from the group consisting of aluminum (Al), beryllium (Be), manganese (Mn), scandium (Sc), yttrium (Y), lanthanum (La), and cerium (Ce).

For example, the metal electrode may be a formed article made of the first metal, an alloy of these, or the like. Alternatively, the metal electrode may be one obtained by coating a formed article made of another metal or alloy, or a carbon material with the first metal. Examples of the further metal include Ag, Cu, Ni, Pb, Hg, Tl, Bi, In, Sn, Cd, Au, Zn, Pd, Ga, Ge, Fe, Pt, Pd, Ru, Cr, Mo, W, V, Nb, Ta, and Zr. Examples of the above carbon material include glassy carbon (GC), natural graphite (graphite), isotropic carbon, highly oriented pyrolytic graphite (HOPG), plastic formed carbon (PFC), and conductive diamond.

(iii) Application of voltage (S13)

Subsequently, a voltage is applied to the molten salt. Accordingly, $CO_3^{2-}$ is reduced on the metal electrode to produce carbon (Equation 1). The produced carbon reacts with the first metal contained in the metal electrode to produce a carbide of the first metal. The carbide of the first metal is precipitated on the cathode. When the first metal is Al, aluminum carbide is obtained (Equation 2).

$$\text{(Equation 1)} \qquad CO_3^{2-} + 4e^- \rightarrow C + 3O^{2-}$$

$$\text{(Equation 2)} \qquad 3C + 4Al \rightarrow Al_4C_3$$

$O^{2-}$ is oxidized on the anode to produce oxygen (Equation 3). The oxygen generated on the anode is discharged into the gas phase. This oxygen gas can be recovered and utilized in other applications.

$$\text{(Equation 3)} \qquad 2O^{2-} \rightarrow O_2 + 4e^-$$

When the molten salt contains a calcium ion, metallic calcium may be further produced on the cathode (Equation 4). A part or the whole metallic calcium produced by this side reaction may be further reacted to be calcium carbide (Equation 5). Alternatively, metallic calcium may be reacted with carbon dioxide physically dissolved in the molten salt to be calcium carbide (Equation 6). Calcium carbide may be precipitated on the cathode. Fine carbon powder may be produced, so that the molten salt may be turbid in black, in some cases (Equation 7). CaO produced in the above (Equation 6) and (Equation 7) is immediately dissolved in the molten salt and produces calcium ions and oxide ions (Equation 8).

$$\text{(Equation 4)} \qquad Ca^{2+} + 2e^- \rightarrow Ca$$

$$\text{(Equation 5)} \qquad Ca + 2C \rightarrow CaC_2$$

$$\text{(Equation 6)} \qquad 2CO_2 + 5Ca \rightarrow CaC_2 + 4CaO$$

$$\text{(Equation 7)} \qquad 2Ca + CO_2 \rightarrow C + 2CaO$$

$$\text{(Equation 8)} \qquad CaO \rightarrow Ca^{2+} + O^{2-}$$

When the first metal is beryllium, manganese, scandium, yttrium, lanthanum, or cerium, a carbide of the metal is precipitated on the cathode by the same reaction.

The application of a voltage is carried out at a temperature at which the molten state of the molten salt can be maintained, that is, a temperature about 10°C or higher than the melting point of the molten salt. The temperature of the electrolytic bath may be, for example, 510°C or more, or 550°C or more, in the case of a NaCl-KC1 eutectic salt (melting point: 503.8°C). The temperature of the electrolytic bath may be, for example, 800°C or less, or 700°C or less. According to the present disclosure, the reaction proceeds at such a relatively low temperature, so that the energy efficiency is high.

The applied voltage is set so that the cathode potential can be a potential lower than the potential (Ec) at which carbonate ions are discharged on the metal electrode. Accordingly, the selectivity of a first metal carbide may be further improved. When the potential of the cathode is excessively high, the target first metal carbide is hardly produced. When the potential of the cathode is excessively low, a metal having an oxidation reduction potential in the molten salt higher than the set cathode potential among metals contained in the molten salt is also precipitated, while the first metal carbide is also produced. When a plurality of metals each having a similar oxidation reduction potential in the molten salt is present in the molten salt, an alloy of a plurality of metals may be precipitated depending on the set cathode potential. The potential Ec can be determined using the metal electrode in the molten salt to be used by carrying out cyclic voltammetry measurement in the molten salt. The cathode potential is a value obtained by measuring the potential between the reference electrode (Ag+/Ag) and the cathode, and calibrating the measured value based on the metal precipitation potential. The metal precipitation potential is the precipitation potential of a Na-Ca alloy, in the case of the NaCl-KCl eutectic salt.

The set current value when constant current electrolysis is carried out may be appropriately set so that the cathode potential during electrolysis can be the above-described potential range.

In the first aspect as one aspect of the present disclosure, a method for producing a metal carbide includes:

preparing an electrolytic bath containing a molten salt containing at least one selected from the group consisting of a sodium ion, a lithium ion, and a potassium ion, and at least one selected from the group consisting of a calcium ion, a magnesium ion, a strontium ion, and a barium ion, as metal ions;
adding gas containing carbon dioxide to the electrolytic bath to prepare a molten salt containing a carbonate ion;
preparing an electrode containing at least one first metal selected from the group consisting of aluminum, beryllium, manganese, scandium, yttrium, lanthanum, and cerium; and
applying a voltage to the molten salt using the electrode containing the first metal to obtain a metal carbide composition containing a carbide of the first metal.

In the second aspect as one aspect of the present disclosure, a method for producing a metal carbide includes:

preparing an electrolytic bath containing a molten salt containing a sodium ion and a calcium ion as metal ions;
adding gas containing carbon dioxide to the electrolytic bath to prepare a molten salt containing a carbonate ion;
preparing a working electrode containing aluminum as the first metal, a counter electrode containing platinum, and a reference electrode containing silver; and
applying a voltage to the molten salt for 30 minutes using the working electrode containing aluminum, while maintaining the potential of the working electrode with respect to the reference electrode at 0.08 to 0.17 V to obtain a metal carbide composition containing aluminum carbide.

In the third aspect as one aspect of the present disclosure, a method for producing a metal carbide includes:

preparing an electrolytic bath containing a molten salt containing a sodium ion and a calcium ion as metal ions;
blowing carbon dioxide into the electrolytic bath containing the molten salt heated at 600°C at a flow rate of 100 mL/min for 60 min or more to prepare a molten salt containing a carbonate ion;
preparing a working electrode containing aluminum as the first metal, a counter electrode containing platinum, and a reference electrode containing silver; and
applying a voltage to the molten salt for 30 minutes using the working electrode containing aluminum, while maintaining the potential of the working electrode with respect to the reference electrode at 0.08 to 0.17 V to obtain a metal carbide composition containing aluminum carbide.

In the fourth aspect as one aspect of the present disclosure, a method for producing a metal carbide includes:

preparing an electrolytic bath containing a molten salt containing at least one selected from the group consisting of a sodium ion, a lithium ion, and a potassium ion, at least one selected from the group consisting of a calcium ion, a magnesium ion, a strontium ion, and a barium ion, and a carbide of at least one first metal selected from the group consisting of aluminum, beryllium, manganese, scandium, yttrium, lanthanum, and cerium, as metal ions;
adding gas containing carbon dioxide to the electrolytic bath to prepare a molten salt containing a carbonate ion;
preparing an electrode containing at least one first metal selected from the group consisting of aluminum, beryllium, manganese, scandium, yttrium, lanthanum, and cerium; and
applying a voltage to the molten salt using the electrode containing the first metal to obtain a metal carbide composition containing a carbide of the first metal.

In the fourth aspect, the first metal constituting the carbide contained in the molten salt may be the same metal as the first metal contained in the electrode.

In the fourth aspect, the content of the carbide of the first metal in the molten salt may be 0.8 to 6.0 mol%, or may be 0.9 to 5.3 mol% per 100 mol% of the molten salt.

In the fifth aspect as one aspect of the present disclosure, a method for producing a metal carbide includes:

preparing an electrolytic bath containing a molten salt containing a sodium ion, a calcium ion, and aluminum carbide, as metal ions;

adding gas containing carbon dioxide to the electrolytic bath to prepare a molten salt containing a carbonate ion;
preparing a working electrode containing aluminum as the first metal, a counter electrode containing platinum, and a reference electrode containing silver; and
applying a voltage to the molten salt for 5 to 15 minutes using the working electrode containing aluminum, while maintaining the potential of the working electrode with respect to the reference electrode at 0.15 to 0.45 V to obtain a metal carbide composition containing aluminum carbide.

In the sixth aspect as one aspect of the present disclosure, a method for producing a metal carbide includes:

preparing an electrolytic bath containing a molten salt containing a sodium ion, a calcium ion, and aluminum carbide, as metal ions;
blowing carbon dioxide into the electrolytic bath containing the molten salt heated at 600°C at a flow rate of 100 mL/min for 60 min or more to prepare a molten salt containing a carbonate ion;
preparing a working electrode containing aluminum as the first metal, a counter electrode containing platinum, and a reference electrode containing silver; and
applying a voltage to the molten salt for 5 to 15 minutes using the working electrode containing aluminum, while maintaining the potential of the working electrode with respect to the reference electrode at 0.15 to 0.45 V to obtain a metal carbide composition containing aluminum carbide.

In the fifth to sixth aspects, the content of aluminum carbide in the molten salt may be 0.8 to 6.0 mol%, or 0.9 to 5.3 mol% per 100 mol% of the molten salt.

(Metal carbide composition)

The metal carbide composition contains a carbide of the first metal. The first metal carbide is the main component of the metal carbide composition. The main component refers to a component that accounts for 50% by mass or more of the total mass of the metal carbide composition. The content of the first metal carbide may be 80% by mass or more, or 90% by mass or more of the mass of the metal carbide composition. The content of the first metal carbide may be 99.9% by mass or less, or 99% by mass or less of the mass of the metal carbide composition. In one aspect, the content of the first metal carbide is 80% by mass or more and 99.9% by mass or less of the mass of the metal carbide composition.

The metal carbide composition is usually obtained in a state of being supported on the cathode (strictly, a base derived from the metal electrode used as the cathode). When a molten salt in which the solubility of the first metal carbide is high is used, the first metal carbide can be obtained in a state where a part or the whole thereof is dissolved in the molten salt. When the first metal carbide is dissolved in the molten salt in advance, the first metal carbide obtained by electrolysis is suppressed to be dissolved in the molten salt and is easily obtained in a state of being supported on a base derived from the metal electrode.

The metal carbide composition may contain at least one selected from the group consisting of carbon; a simple substance, a halide, a carbonate, an oxide, a hydride, and a peroxide of the first metal. The metal carbide composition may further contain at least one selected from the group consisting of a simple substance, a halide, a carbonate, an oxide, and a carbide of the second metal.

The metal carbide composition may further contain at least one selected from the group consisting of a solidified electrolyte, a halide of a material constituting the apparatus, an oxide, a metal, and a hydrate thereof.

The carbon contained in the metal carbide composition is, for example, at least one selected from the group consisting of nanocarbon materials such as graphite, amorphous carbon, glassy carbon, carbon nanotubes, diamond, nanodiamond, and graphene.

The presence confirmation and the quantitative determination of the first metal carbide, the single body of the first metal, the compound containing the first metal, and other impurities can be carried out by, for example, Raman spectroscopy and X-Ray diffraction (XRD) analysis of the composition.

[Metal-containing member]

The metal-containing member according to the present embodiment includes a base containing a first metal, and a metal carbide composition supported on the base, the metal carbide composition containing a carbide of the first metal. Such a metal-containing member can be utilized in the production of a hydrocarbon.

The metal-containing member is obtained by, for example, the above method for producing a metal carbide. That is, the metal-containing member may correspond to the metal electrode after electrolysis of the above molten salt using the metal electrode. In this case, the base is derived from the above metal electrode.

The metal carbide composition is only required to be supported on at least a part of the surface of the base. The surface of the base typically corresponds to the part that has been in contact with the carbonate ion of the metal electrode. The term supported includes a state in which at least a part of the surface of the base is coated with the metal carbide composition.

When a cross section passing through the center (or center of gravity) of the metal-containing member is subjected to elemental analysis with an energy dispersive X-ray analysis (EDX), the first metal and carbon are detected on the cross section. When the first metal and carbon are detected in a mixed state, it can be said that the first metal carbide is present.

[Method for producing hydrocarbon]

The method for producing a hydrocarbon according to the present embodiment includes preparing a molten salt containing a carbonate ion derived from carbon dioxide, preparing an electrode containing the first metal (metal electrode), applying a voltage to the molten salt using the metal electrode to obtain a metal carbide composition containing a carbide of the first metal, and hydrolyzing the carbide of the first metal to obtain gas containing a hydrocarbon. In this method, hydrocarbon is synthesized using $CO_2$ as a raw material, resulting in a contribution to decarbonization.

(1) Preparation of molten salt (S21)

A molten salt is prepared in the same manner as in preparation of molten salt (S11) in the above method for producing a metal carbide.

(2) Preparation of electrode containing first metal (S22)

A metal electrode is prepared in the same manner as in preparation of electrode containing first metal (S12) in the above method for producing a metal carbide.

(3) Application of voltage (S23)

A voltage is applied to the molten salt in the same manner as in application of voltage (S13) in the above method for producing a metal carbide. Consequently, a composition containing the first metal carbide is obtained.

(4) Hydrolysis of metal carbide (S24)

Then, the first metal carbide is brought into contact with water and hydrolyzed. Consequently, gas containing the target hydrocarbon is obtained. Usually, a hydrocarbon has a low solubility in water. Thus, the produced hydrocarbon is rapidly discharged into the gas phase and recovered.

The first metal carbide may be isolated from the carbide composition of a metal and hydrolyzed. Isolation is conducted by, for example, a method in which the carbide composition of a metal is pulverized and a difference in specific gravity is utilized. Alternatively, the carbide composition of a metal may be hydrolyzed as it is. For example, the electrode on which the metal carbide composition is precipitated (it may be the "metal-containing member" according to the present disclosure) is brought into contact with water as it is. In this case, the carbide of the second metal that may be contained in the carbide composition of a metal is also hydrolyzed to produce a hydrocarbon.

Examples of the hydrocarbon to be obtained include methane ($CH_4$), ethane, ethylene, acetylene ($C_2H_2$), methylacetylene, propane, propylene, butane, and butene. In the case where the isolated first metal carbide is used or the amount of impurities (in particular, a simple substance of metal) contained in the composition is small, methane is obtained as the main component. The main component refers to a component that accounts for 50% by mass or more of the total mass of the recovered gas. Methane is the main component of natural gas and utilized as city gas.

The gas to be obtained may contain water vapor, hydrogen, nitrogen, and oxygen as impurities, in addition to the hydrocarbon. The amount of impurities is preferably 10% by mass or less, and more preferably 1% by mass or less of the recovered gas. The amount of impurities may be 0.0001% by mass or more, or 0.001% by mass or more of the recovered gas. In an aspect, the amount of impurities is 0.0001% by mass or more and 1% by mass or less of the recovered gas.

The gas to be obtained may contain methane, and may further contain at least one selected from the group consisting of ethylene, ethane, acetylene, methylacetylene, propylene, butene, and hydrogen.

The presence confirmation and the quantitative determination of hydrocarbon and impurities can be carried out by, for example, gas chromatography (GC analysis), mass spectrometry (MS analysis), gas chromatography mass spectrometry (GC-MS analysis), Fourier transform infrared spectroscopy (FT-IR analysis) with gas cells, or ultraviolet-visible absorption spectroscopy (UV-Vis analysis).

The amount of water to be brought into contact with the composition is appropriately set depending on the mass of the composition. The above amount of water is, for example, more than the amount required for the hydrolysis of the metal carbide and metal contained in the composition. In addition, an amount of water that enables the entire composition to be immersed and takes the evaporation due to the heat generated upon hydrolysis into consideration is desirably used.

The hydrolysis of the first metal carbide produces the hydroxide of the first metal together with the hydrocarbon. For example, the hydrolysis of aluminum carbide produces aluminum hydroxide together with methane (Equation 9).

$$\text{(Equation 9)} \qquad Al_4C_3 + 12H_2O \rightarrow 3CH_4 + 4Al(OH)_3$$

Hereinabove, the embodiments of the present disclosure have been described in detail, but the present disclosure is not limited to these, and its design can be modified within a range not departing from the gist of the present disclosure.

In the above embodiments, carbon dioxide is used as the carbonate ion source, but the carbonate ion source is not limited thereto. The carbonate ion source may be a carbonate of an arbitrary metal. When the carbonate of the second metal is used, the second metal ion and carbonate ions are generated by ionization. The carbonate of the second metal can be synthesized by, for example, reacting the hydroxide of the second metal with carbon dioxide.

In the above embodiment, the carbon-containing member to be obtained includes a base, and a metal carbide composition containing the carbide of the first metal supported on the base. The carbon-containing member to be obtained by the method shown in the present embodiment may include a base, a metal carbide layer, and a carbon layer. The carbide of the first metal may be hydrolyzed while being reacted with water and the moisture in the atmosphere to produce hydrocarbon (e.g., Equation 9). The above hydrolysis is suppressed by providing a carbon layer on the outer side of the metal carbide layer.

EXAMPLES

Hereinafter, the present invention will be more specifically described with reference to Examples, but the present invention is not limited thereto.

[Example 1]

(Production of metal carbide)

8.0 mol% of CaO with respect to NaCl, and $CaCl_2$ ($NaCl/CaCl_2$ = 47.9 mol%/52.1 mol%) having an eutectic composition was mixed thereinto, and the mixture was dried in vacuum at 200°C and 100 Pa or less for 24 hours or more. This mixed salt was put in a container made of alumina, set in an electric furnace, and heated to 600°C. Thus, a molten salt of $NaCl\text{-}CaCl_2\text{-}CaO$ was obtained.

Then, a working electrode (1 cm × 1.5 cm, Al), a counter electrode (1 cm × 1.5 cm or more, a platinum plate), and a reference electrode ($Ag^+/Ag$) were attached to the lid of the above container, and the container was sealed with this lid. $CO_2$ was blown into the molten salt at 600°C in the container at a flow rate of 100 mL/min for 60 minutes or more. Subsequently, a voltage was applied for 30 minutes while maintaining the potential of the working electrode relative to the reference electrode at 0.08 V using a potentio-galvanostat. A precipitate was confirmed to be precipitated on the working electrode. All the experimental operations were carried out in a glove box in which a high purity argon atmosphere was maintained.

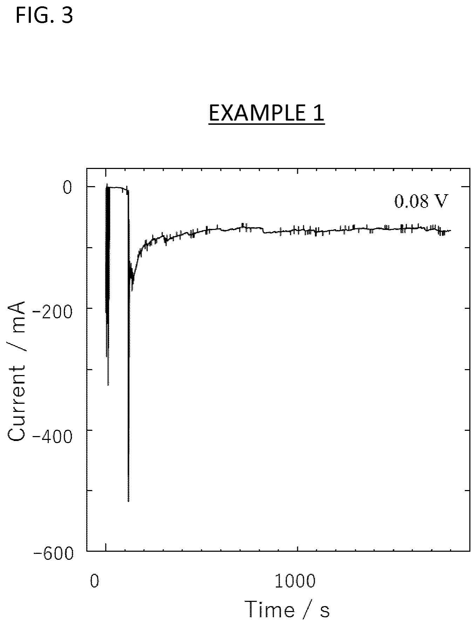

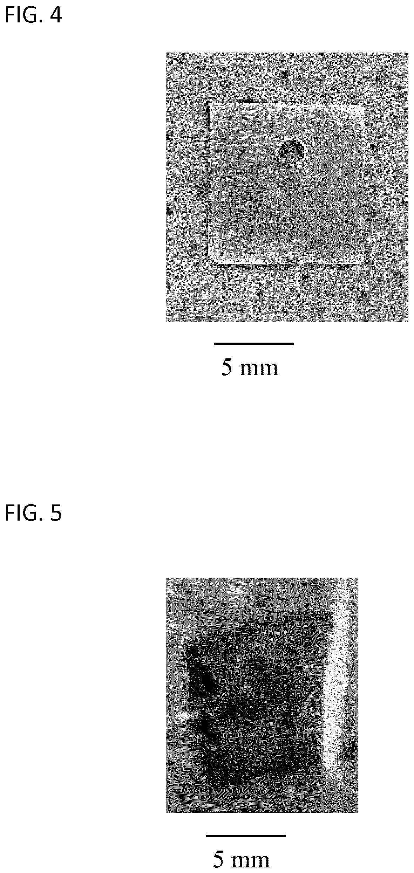

FIG. 3 shows a change in the potential of the working electrode with respect to the reference electrode. FIG. 4 shows the appearance of the working electrode before energization. FIG. 5 shows the appearance of the working electrode after energization. A black precipitate was confirmed on a surface of the working electrode.

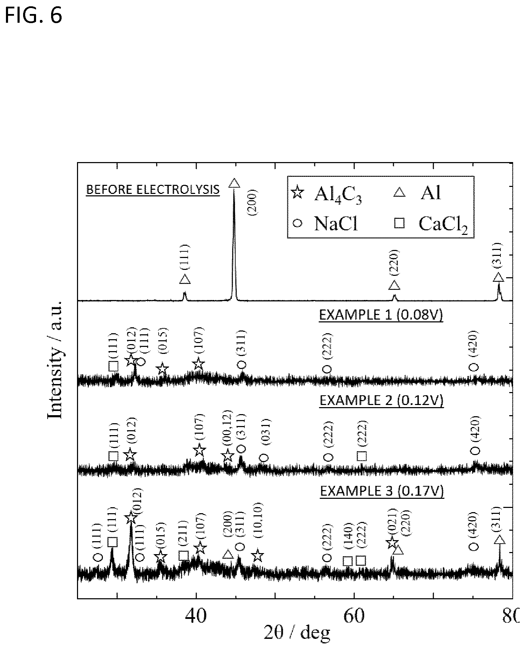

It was confirmed from the XRD analysis of the obtained precipitate that $Al_4C_3$ is contained, and at least NaCl, $CaCl_2$, and carbon are contained as impurities in the precipitate. FIG. 6 shows the results of the XRD analysis of the obtained precipitate. The mass proportion of impurities in the precipitate was sufficiently smaller than 50% by mass. FIG. 6 shows the results of the analysis of the working electrode before application of a voltage (before electrolysis) and the results of the analysis of the precipitate obtained in Examples 1 to 3 together.

(Preparation of hydrocarbon)

The precipitate was put in a sealed test tube. Pure water was added to the test tube in small portions at ambient temperature (23°C), and the precipitate was hydrolyzed. The total amount of the water added was 2.5 ml. After bubbling was confirmed to be generated in the test tube, the test tube was allowed to stand until no bubbling was observed. Subsequently, 100 µl (microliter) of the gas in the test tube was collected using a gas tight syringe.

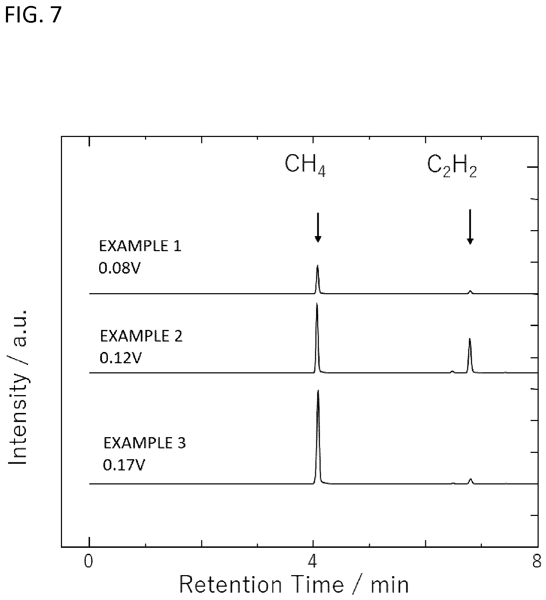

The obtained gas was subjected to GC-MS analysis using a gas chromatograph (GC) apparatus, and the

production of $CH_4$ as the main component was confirmed. Further, acetylene and hydrogen were confirmed to be produced as byproducts. In addition, water, carbon dioxide, nitrogen, oxygen, and argon were contained. The amount of each component produced was also confirmed. The mass proportion of $CH_4$ in the recovered gas was sufficiently larger than 90% by mass. FIG. 7 shows the results of the GC analysis.

With respect to $CH_4$ gas production, the faradaic efficiency was calculated to be about 1.2%. It can be said that the higher the faradaic efficiency with respect to $CH_4$ gas production is, the higher the faradaic efficiency with respect to aluminum carbide production is. With respect to $C_2H_2$ gas production, the faradaic efficiency was calculated to be about 0.032%. It can be said that the higher the faradaic efficiency with respect to $C_2H_2$ gas production is, the higher the faradaic efficiency with respect to calcium carbide production is.

[Example 2]

The precipitate and hydrocarbon were obtained in the same manner as in Example 1, except that a voltage was applied for 30 minutes while maintaining the potential of the working electrode with respect to the reference electrode at 0.12 V.

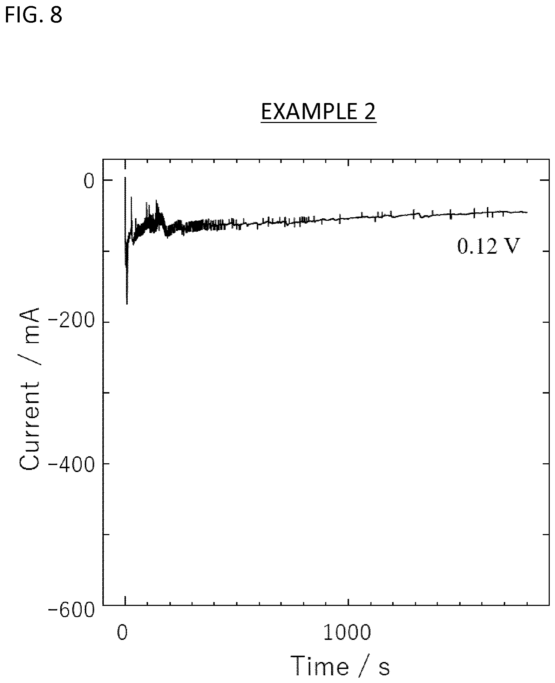

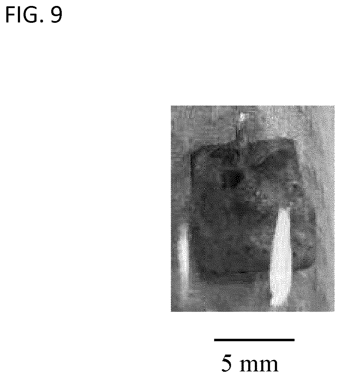

FIG. 8 shows a change in the potential of the working electrode with respect to the reference electrode. FIG. 9 shows the appearance of the working electrode after energization. A black precipitate was confirmed on a surface of the working electrode. It was confirmed from the XRD analysis of the obtained precipitate that $Al_4C_3$ is contained, and at least NaCl, $CaCl_2$, and carbon are contained as impurities in the precipitate. FIG. 6 shows the results of the XRD analysis of the obtained precipitate. The mass proportion of impurities in the precipitate was sufficiently smaller than 50% by mass.

The obtained gas was subjected to GC-MS analysis using a gas chromatograph (GC) apparatus, and the production of $CH_4$ as the main component was confirmed. Further, acetylene and hydrogen were confirmed to be produced as byproducts. In addition, water, carbon dioxide, nitrogen, oxygen, and argon were contained. The amount of each component produced was also confirmed. With respect to $CH_4$ gas production, the faradaic efficiency was calculated to be about 8.4%. With respect to $C_2H_2$ gas production, the faradaic efficiency was calculated to be about 1.2%. The mass proportion of $CH_4$ in the recovered gas was sufficiently larger than 90% by mass. FIG. 7 shows the results of the GC analysis.

[Example 3]

The precipitate and hydrocarbon were obtained in the same manner as in Example 1, except that a voltage was applied for 30 minutes while maintaining the potential of the working electrode with respect to the reference electrode at 0.17 V.

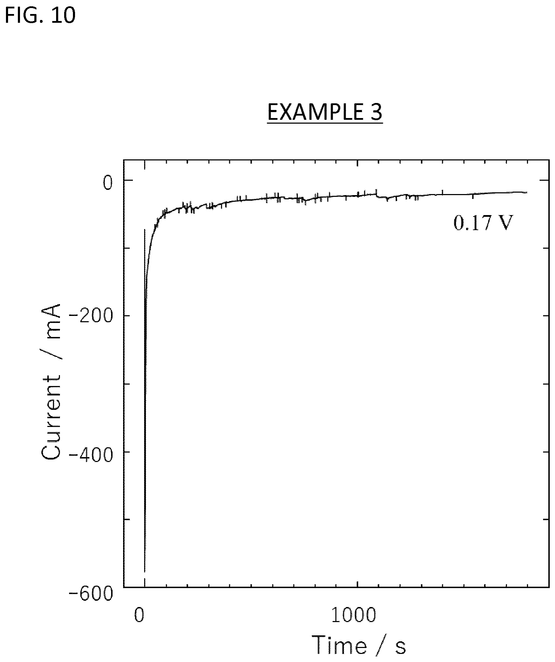

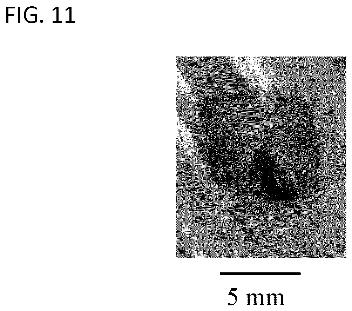

FIG. 10 shows a change in the potential of the working electrode with respect to the reference electrode. FIG. 11 shows the appearance of the working electrode after energization. A black precipitate was confirmed on a surface of the working electrode. It was confirmed from the XRD analysis of the obtained precipitate that $Al_4C_3$ is contained, and at least NaCl, $CaCl_2$, and carbon are contained as impurities in the precipitate. FIG. 6 shows the results of the XRD analysis of the obtained precipitate. The mass proportion of impurities in the precipitate was sufficiently smaller than 50% by mass.

The obtained gas was subjected to GC-MS analysis using a gas chromatograph (GC) apparatus, and the production of $CH_4$ as the main component was confirmed. Further, acetylene, ethane, and hydrogen were confirmed to be produced as byproducts. In addition, water, carbon dioxide, nitrogen, oxygen, and argon were contained. The amount of each component produced was also confirmed. With respect to $CH_4$ gas production, the faradaic efficiency was calculated to be about 14%. With respect to $C_2H_2$ gas production, the faradaic efficiency was calculated to be about 0.19%. The mass proportion of $CH_4$ in the recovered gas was sufficiently larger than 50% by mass. FIG. 7 shows the results of the GC analysis.

[Examples 4 to 7]

The precipitate and hydrocarbon were obtained in the same manner as in Example 1, except that 8.0 mol% of CaO with respect to NaCl and $CaCl_2$ (NaCl/$CaCl_2$ = 47.9 mol%/52.1 mol%) having an eutectic composition was mixed and completely melted, 6.0 mol% of $Al_4C_3$ was then further mixed therewith (the content of $Al_4C_3$: about 5.3 mol% per 100 mol% of the molten salt), and a voltage was applied for 5 minutes while maintaining each potential of the working electrode with respect to the reference electrode at 0.45 V, 0.35 V, 0.25 V, and 0.15 V.

[Examples 8 to 11]

The precipitates and hydrocarbons were obtained in the same manner as in Examples 4 to 7, except that a voltage was applied for 10 minutes while maintaining each potential of the working electrode with respect to the reference electrode at 0.45 V, 0.35 V, 0.25 V, and 0.15 V.

[Examples 12 to 15]

The precipitates and hydrocarbons were obtained in the same manner as in Examples 4 to 7, except that a voltage was applied for 15 minutes while maintaining each potential of the working electrode with respect to the reference electrode at 0.45 V, 0.35 V, 0.25 V, and 0.15 V.

[Examples 16 to 19]

The precipitates and hydrocarbons were obtained in the same manner as in Examples 4 to 7, except that the added amount of $Al_4C_3$ was changed to 1 mol% (the content of $Al_4C_3$: about 0.9 mol% per 100 mol% of the molten salt).
The amounts of $CH_4$ and $C_2H_2$ produced obtained in Examples 1 to 19 are shown in Table 1 together. In Table 1, the amount of $Al_4C_3$ (mol%) is described as a proportion when the total amount of NaCl and $CaCl_2$ is defined as 100 mol%.

[Table 1]

| Example | $Al_4C_3$ (mol%) | Electrolysis potential (V) | Electrolysis time (min) | Faradaic efficiency(%) | |
|---|---|---|---|---|---|
| | | | | $CH_4$ | $C_2H_2$ |
| 1 | 0 | 0.08 | 30 | 1.2 | 0.032 |
| 2 | 0 | 0.12 | 30 | 8.4 | 1.2 |
| 3 | 0 | 0.17 | 30 | 14 | 0.19 |
| 4 | 6 | 0.45 | 5 | 66 | 0.44 |
| 5 | 6 | 0.35 | 5 | 24 | 0.43 |
| 6 | 6 | 0.25 | 5 | 11 | 0.23 |
| 7 | 6 | 0.15 | 5 | 7.8 | 0.22 |
| 8 | 6 | 0.45 | 10 | 37 | 0.68 |
| 9 | 6 | 0.35 | 10 | 33 | 0.41 |
| 10 | 6 | 0.25 | 10 | 7.8 | 0.53 |
| 11 | 6 | 0.15 | 10 | 12 | 0.78 |
| 12 | 6 | 0.45 | 15 | 16 | 0.12 |
| 13 | 6 | 0.35 | 15 | 29 | 0.35 |
| 14 | 6 | 0.25 | 15 | 11 | 0.96 |
| 15 | 6 | 0.15 | 15 | 11 | 1.2 |
| 16 | 1 | 0.45 | 5 | 3.7 | 0 |
| 17 | 1 | 0.35 | 5 | 16 | 0.014 |
| 18 | 1 | 0.25 | 5 | 3.3 | 1.0 |
| 19 | 1 | 0.15 | 5 | 2.0 | 2.0 |

It was confirmed from these results that the faradaic efficiency is improved by including $Al_4C_3$ in the molten salt.
The faradaic efficiency e with respect to $CH_4$ production was calculated as follows.
First, the volume proportion of $CH_4$ contained in the recovered gas was calculated based on the total area of the peaks and the calibration curve obtained by GC-MS analysis. Then, the volume of $CH_4$ produced was calculated based on the volume occupied by the gas phase in the recovery container and the volume proportion of $CH_4$ in the calculated gas. Finally, the faradaic efficiency e (%) was calculated by the following expression assuming that the $CH_4$ produced was in standard conditions (0°C, 101 kPa).

[Expression 1]

$$e\,[\%] = \frac{\text{Actually measured amount of CH4 produced [mol]}}{\text{Theoretical amount of CH4 produced, determined from electrical quantity [mol]}} \times 100$$

$$= \frac{\dfrac{\text{Calculated volume of CH4 produced [L]}}{\text{Volume of CH4 in standard state (22.4) [L/mol]}}}{\dfrac{\text{Average current value during electrolysis [A]} \times \text{electrolysis time [s]}}{\text{Faraday constant (96485) [C/mol]} \times \text{Number of electrons in CH4 (10) [$-$]}}} \times 100$$

INDUSTRIAL APPLICABILITY

The production method of the present disclosure is useful in various fields because it enables the reaction to rapidly proceed at a relatively low temperature and a metal carbide to be efficiently obtained without requiring use of a flammable raw material.

This present application claims the benefit of priority based on Japanese Patent Application Nos.2024-028876 filed on February 28, 2024 and 2024-204704 filed on November 25, 2024, the entire contents of which are incorporated herein by reference.

**Claims**

1.  A method for producing a metal carbide, comprising:

    preparing a molten salt containing a carbonate ion;
    preparing an electrode containing a first metal; and
    applying a voltage to the molten salt using the electrode to obtain a metal carbide composition containing a carbide of the first metal.

2.  The method for producing a metal carbide according to claim 1, wherein the first metal contains at least one selected from the group consisting of aluminum, beryllium, manganese, scandium, yttrium, lanthanum, and cerium.

3.  The method for producing a metal carbide according to claim 1 or 2, wherein the molten salt contains at least one selected from the group consisting of a sodium ion, a lithium ion, a potassium ion, rubidium, and cesium, as a metal ion.

4.  The method for producing a metal carbide according to any one of claims 1 to 3, wherein the molten salt contains at least one selected from the group consisting of a sodium ion, a lithium ion, and a potassium ion, and at least one selected from the group consisting of a calcium ion, a magnesium ion, a strontium ion, and a barium ion, as metal ions.

5.  The method for producing a metal carbide according to any one of claims 1 to 4, wherein the molten salt contains the carbide of the first metal.

6.  The method for producing a metal carbide according to any one of claims 1 to 5, wherein the carbide composition further contains at least one selected from the group consisting of carbon; a simple substance, a halide, a carbonate, an oxide, a hydride, and a peroxide of the first metal; and a simple substance, a halide, a carbonate, an oxide, and a carbide of the second metal constituting the molten salt.

7.  A method for producing a hydrocarbon, comprising:

    preparing a molten salt containing a carbonate ion;
    preparing an electrode containing a first metal;
    applying a voltage to the molten salt using the electrode to obtain a metal carbide composition containing a carbide of the first metal; and
    hydrolyzing the carbide of the first metal to obtain gas containing a hydrocarbon.

8.  The method for producing a hydrocarbon according to claim 7, wherein the gas contains methane.

9. The method for producing a hydrocarbon according to claim 7 or 8, wherein the gas contains methane and at least one selected from the group consisting of ethylene, ethane, acetylene, methylacetylene, propylene, butene, and hydrogen.

10. A metal-containing member comprising:

a base containing a first metal; and
a metal carbide composition supported on the base, wherein the metal carbide composition contains a carbide of the first metal.

FIG. 1

```
                    ┌─────────────┐
                    │    START    │
                    └──────┬──────┘
                           │
                           ▼
        ┌───────────────────────────────────┐
        │  PREPARATION OF FIRST MOLTEN       │─── S11
        │  SALT CONTAINING CARBONATE         │
        │  ION                               │
        └──────────────────┬────────────────┘
                           │
                           ▼
        ┌───────────────────────────────────┐
        │  PREPARATION OF FIRST              │─── S12
        │  ELECTRODE CONTAINING FIRST        │
        │  METAL                             │
        └──────────────────┬────────────────┘
                           │
                           ▼
        ┌───────────────────────────────────┐
        │  APPLICATION OF VOLTAGE            │─── S13
        │  (PRODUCTION OF FIRST METAL        │
        │  CARBIDE)                          │
        └──────────────────┬────────────────┘
                           │
                           ▼
                    ┌─────────────┐
                    │     END     │
                    └─────────────┘
```

FIG. 2

START

PREPARATION OF FIRST MOLTEN SALT CONTAINING CARBONATE ION — S21(S11)

PREPARATION OF FIRST ELECTRODE CONTAINING FIRST METAL — S22(S12)

APPLICATION OF VOLTAGE (PRODUCTION OF FIRST METAL CARBIDE) — S23(S13)

HYDROLYSIS OF FIRST METAL CARBIDE (PRODUCTION OF HYDROCARBON) — S24

END

FIG. 3

EXAMPLE 1

FIG. 4

5 mm

FIG. 5

5 mm

FIG. 6

FIG. 7

FIG. 8

EXAMPLE 2

FIG. 9

5 mm

FIG. 10

EXAMPLE 3

FIG. 11

5 mm

## INTERNATIONAL SEARCH REPORT

| International application No. |
|---|
| **PCT/JP2025/007004** |

### A. CLASSIFICATION OF SUBJECT MATTER

*C25B 1/01*(2021.01)i; *C01B 32/914*(2017.01)i; *C07C 1/32*(2006.01)i; *C07C 9/04*(2006.01)i; *C07C 9/06*(2006.01)i; *C07C 11/04*(2006.01)i; *C07C 11/06*(2006.01)i; *C07C 11/08*(2006.01)i; *C07C 11/22*(2006.01)i; *C07C 11/24*(2006.01)i; *C25B 1/02*(2006.01)i; *C25B 1/135*(2021.01)i; *C25B 9/00*(2021.01)i; *C25B 9/09*(2021.01)i
FI: C25B1/01 Z; C25B9/09; C07C1/32; C07C9/04; C07C9/06; C07C11/04; C07C11/06; C07C11/08; C07C11/24; C07C11/22; C25B1/135; C25B1/02; C25B9/00 Z; C01B32/914

According to International Patent Classification (IPC) or to both national classification and IPC

### B. FIELDS SEARCHED

Minimum documentation searched (classification system followed by classification symbols)

C25B1/01; C01B32/914; C07C1/32; C07C9/04; C07C9/06; C07C11/04; C07C11/06; C07C11/08; C07C11/22; C07C11/24; C25B1/02; C25B1/135; C25B9/00; C25B9/09

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | LIANG, Xinxin et al. Electrochemical Reduction of Carbon Dioxide and Iron Oxide in Molten Salts to Fe/Fe3C Modified Carbon for Electrocatalytic Oxygen Evolution. Angewante Chemie. 18 January 2021, vol. 133, no. 4, pp. 2148-2152, 2148, right column, lines 14-40, fig. 1 | 1, 3, 6, 10 |
| A | | 2, 4, 5, 7-9 |
| X | JP 2006-169554 A (DOSHISHA) 29 June 2006 (2006-06-29) claims, paragraphs [0027], [0032], examples, fig. 1 | 1-4, 6, 10 |
| A | | 5, 7-9 |
| X | JP 2023-146133 A (IMSEP CO., LTD.) 12 October 2023 (2023-10-12) claims, paragraphs [0036]-[0041], [0048], drawings | 1-4, 6, 10 |
| A | | 5, 7-9 |

☑ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **21 May 2025** | **03 June 2025** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**

International application No.

**PCT/JP2025/007004**

| C. | DOCUMENTS CONSIDERED TO BE RELEVANT | |
|---|---|---|
| Category* | Citation of document, with indication, where appropriate, of the relevant passages | Relevant to claim No. |
| A | JP 2015-232171 A (IMSEP CO., LTD.) 24 December 2015 (2015-12-24) paragraphs [0002], [0003] | 1-10 |
| A | JP 2023-54787 A (DOSHISHA) 14 April 2023 (2023-04-14) | 1-10 |

Form PCT/ISA/210 (second sheet) (July 2022)

**INTERNATIONAL SEARCH REPORT**
Information on patent family members

International application No.

**PCT/JP2025/007004**

| Patent document cited in search report | | | Publication date (day/month/year) | Patent family member(s) | | | Publication date (day/month/year) |
|---|---|---|---|---|---|---|---|
| JP | 2006-169554 | A | 29 June 2006 | (Family: none) | | | |
| JP | 2023-146133 | A | 12 October 2023 | (Family: none) | | | |
| JP | 2015-232171 | A | 24 December 2015 | (Family: none) | | | |
| JP | 2023-54787 | A | 14 April 2023 | JP | 2023-57158 | A | |
| | | | | JP | 2024-169598 | A | |
| | | | | US | 2024/0286974 | A1 | |
| | | | | WO | 2023/058619 | A1 | |
| | | | | EP | 4394085 | A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1183411 A
- JP 2001058810 A
- JP 2024028876 A
- JP 2024204704 A

**Non-patent literature cited in the description**

- **KIYOSHI ITATANI** ; **AKIRA KISHIOKA**. Some Properties of Aluminum Carbide and Its Related Compounds. *Inorganic Materials*, 1997, vol. 4 (271), 633-641